# EUROPEAN PATENT APPLICATION

(11) **EP 2 530 055 A1**
(43) Date of publication of application: **05.12.2012**
(21) Application number: 11168627.5
(22) Date of filing: 03.06.2011
(51) Int. Cl.: C02F 1/68, C02F 1/76, G01N 33/18, C02F 1/00

(54) **System and method of controlling dosing of a disinfectant into water**

(71) Applicant: SIEMENS AKTIENGESELLSCHAFT, 80333 München (DE)
(72) Inventor: Forstmeier, Dieter, 89331 Burgau (DE)

(57) **Abstract**

The invention relates to a system (10) and a method of controlling dosing of a chlorine dioxide based disinfectant into water, wherein the system (10) comprises, at least one dosing unit (15) for dosing the disinfectant into the water, at least one first sensor (20) adapted to detect a concentration of chlorite in the water and configured to output respective first signals (30) indicative of the concentration of the chlorite, and a controller (25) operably coupled to the at least one first sensor (20) to receive the first signals (30) and operably coupled to the at least one dosing unit (15) to send a control signal (35) to the at least one dosing unit (15) in response to the at least one of the first signals (20) such that the concentration of chlorite does not exceed a chlorite threshold value.

## Description

The present invention relates to a system and a method of controlling dosing of a chlorine dioxide based disinfectant into water.

In order to minimize the risk of infectious diseases, it is customary to disinfect water meant for human use. In special applications such as installations for supplying non-stationary drinking water, or the treatment of water in swimming pools, disinfection is a basic necessity. Chlorine and its species, such as, sodium perchlorite, calcium perchlorite are the most commonly used disinfectants for disinfecting water because they are economical and have good germicidal ability. However, the aforementioned disinfectants react with organic material in the water and produce various by-products of disinfection. For example, the disinfection by-products include chloramines and trihalomethanes (THMs). These by-products are restricted in many places, and especially trihalomethanes, as the same is suspected to be a carcinogen.

Alternatively, chlorine dioxide has been proposed for disinfecting water as the same is effective in the inactivation of the pathogenic organisms and unlike chlorine and its species mentioned above does not produce trihalomethanes. However, chlorine dioxide reacts with water to produce inorganic by-products, such as, chlorite and chlorate. The parameters of the water that influence the formation of chlorite and chlorate in the water, include, concentration of the disinfectant, reaction time, temperature, pH value, and total organic content of the water. These by-products are restricted in many places, as exposure to the same at low levels may lead to hemolytic anemia, while higher levels may result in an increase in methemoglobin. Thus, standards for the concentrations of chlorite and chlorate in water have been established in many countries. For example, World Health Organization (WHO) recommends 0.2 mg/l as an upper limit of concentration of chlorite in drinking water.

It is an object of the invention to provide a system and a method of controlling a concentration of chlorite in water generated due to the addition of a chlorine dioxide based disinfectant into the water.

The above object is achieved by a system and a method of controlling dosing of a chlorine dioxide based disinfectant into water according to claim 1 and claim 10.

Controlling the dosing of the disinfectant into the water responsive to the concentration of chlorite in the water enables in controlling the concentration of chlorite in the water more effectively as the concentration of chlorite in the water is maintained below the chlorite threshold value. The chlorite threshold value can be the concentration of chlorite specified by the Government or a standard organization as the permissible limit. This prevents users of the water from exposure to chlorite above the permissible limits. Additionally, the dosing of disinfectant can be controlled in a continuous manner as the control signal is generated by the controller responsive to the concentration of chlorite in the water. Moreover, as the dosing of the disinfectant can be controlled in a continuous manner, the dosing of the disinfectant into the water is not required to be stopped for controlling the concentration of chlorite in the water.

According to an embodiment, further comprising at least one second sensor adapted to detect a concentration of the disinfectant in the water and configured to output respective second signals indicative of the concentration of the disinfectant, wherein the controller is configured to receive the respective second signals and configured to generate the control signal further responsive to the at least one of the second signals such that the concentration of disinfectant in the water is equal to a modified dosing setpoint value, wherein the modified dosing setpoint value is modified responsive to at least one of the first signals. This enables in efficient control of dosing of the disinfectant into the water by reducing the deviation between the amount of the disinfectant being dosed and the modified dosing setpoint value.

According to another embodiment, the system further comprises one or more additional sensors adapted to detect one or more additional parameters influencing the formation of chlorite in the water. This enables in detecting additional parameters influencing the formation of chlorite.

According to yet another embodiment, wherein the additional parameter includes a temperature and a pH value of the water.

According to yet another embodiment, the controller is further configured to generate a look-up table comprising the detected concentration of chlorite, the concentration of disinfectant in the water, and values of at least one of the additional parameter of a plurality of respective measurement cycles and configured to store the generated look-up table in a memory. This enables in storing the concentration of chlorite in the water based on previously detected parameters influencing the formation of chlorite in the water.

According to yet another embodiment, the controller is further configured to generate the control signal in response to the concentration of disinfectant from the look-up table for the detected concentration of chlorite in the water and value of at least one of the additional parameter such that that the concentration of chlorite does not exceed a chlorite threshold value. This enables in generating the control signal based on the parameters of the water detected previously and thus enables is maintaining the concentration of chlorite in the water within permissible limits based on historical data.

According to yet another embodiment, the controller is configured to generate the control signal in response to a highest concentration of chlorite in the water or an average amount of the concentration of chlorite in the water indicated by the respective first signals provided by two or more of the first sensors at the same time. Highest concentration of chlorite enables in generating the control signal such that the concentration of chlorite will not exceed the threshold value under any circumstances. Additionally, average amount enables in more effective control of dosing of the disinfectant into the water as the dosing of the disinfectant is controlled responsive to the concentration of chlorite detected by multiple sensors.

According to yet another embodiment, the controller is further configured to compute a value of quality of the water as a ratio of the detected concentration of the disinfectant in the water to the concentration of chlorite in the water and provide an output signal indicative of the value of quality of the water.

According to yet another embodiment, the system further comprises a display operably coupled to the controller and configured to receive the output signal and provide a visual indication of the value of quality of the water responsive to the output signal. This provides a visual indication of the quality of the water.

Embodiments of the present invention are further described hereinafter with reference to illustrated embodiments shown in the accompanying drawings, in which:
- FIG 1: illustrates a schematic diagram of a system for controlling dosing of a chlorine dioxide based disinfectant into water according to an embodiment herein,
- FIG 2: illustrates an example of a multidimensional table according to an embodiment herein,
- FIG 3: illustrates an exemplary block diagram of a simplified single feedback closed loop control for controlling the dosing of the disinfectant according to an embodiment herein,
- FIG 4: illustrates a schematic diagram of a water distribution system comprising a plurality of systems for controlling dosing of a chlorine dioxide based disinfectant in water according to an embodiment herein, and
- FIG 5: is a flow diagram illustrating a method of controlling dosing of a chlorine dioxide based disinfectant into water according to an embodiment herein.

Various embodiments are described with reference to the drawings, wherein like reference numerals are used to refer to like elements throughout. In the following description, for purpose of explanation, numerous specific details are set forth in order to provide a thorough understanding of one or more embodiments. It may be evident that such embodiments may be practiced without these specific details.

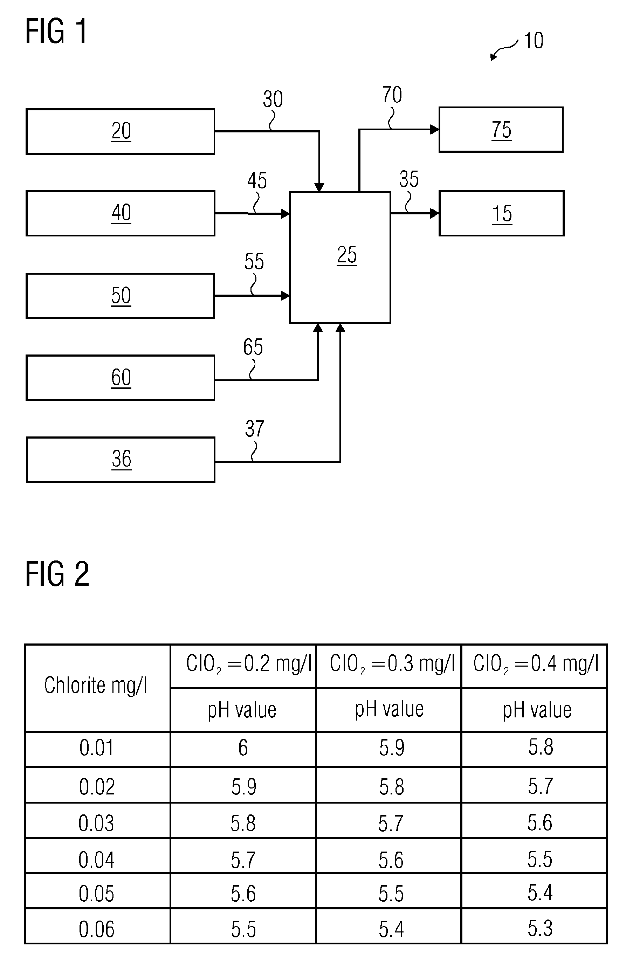

FIG 1 illustrates a schematic diagram of a system 10 for controlling dosing of a chlorine dioxide based disinfectant into water according to an embodiment herein. The system 10 comprises a dosing unit 15 adapted to dose the disinfectant into the water, a first sensor 20 and a controller 25. The dosing unit 15, for example, can be dosing pump. The first sensor 20 is operably coupled to the controller 25 and the controller 25 is operably coupled to the dosing unit 15. The first sensor 20 is adapted to detect a concentration of chlorite in the water and output a first signal 30 indicative of the concentration of chlorite in water. The controller 25 is configured to receive the first signal 30 and is configured to generate a control signal 35 responsive to a value of the first signal 30. The control signal 35 generated by the controller 25 can be provided to the dosing unit 15 and the dosing unit 15 can be configured to operate responsive to the control signal 35. Thus, the dosing of the disinfectant is controlled responsive to the control signal 35. Advantageously, the controller 25 can be configured to generate the control signal 35 such that the amount of disinfectant being dosed into the water by the dosing unit 15 is within a predefined range. The predefined range of dosing of the disinfectant can be stored at a memory and for example, can be selected based on the water quality or type of water being treated. In an aspect, the controller 25 can be configured to compare the value of the first signal 30 with a chlorite threshold value and generate the control signal responsive to the comparison. The chlorite threshold value can be selected as the desired upper limit of concentration of chlorite in the water and the control signal 35 can be generated such that the concentration of chlorite in the water does not exceed the chlorite threshold value. The chlorite threshold value can be stored at a memory internal or external to the controller 25. Thus, the dosing of the disinfectant by the dosing unit 15 is controlled responsive to the concentration of chlorite in the water. This allows for maintaining the concentration of chlorite in the water within the permissible limits more efficiently.

Referring still to FIG 1, in an aspect, the dosing unit 15 can be configured to dose the disinfectant into the water at a rate specified by a dosing setpoint value. The controller 25 can be configured to generate the control signal 35 based on the dosing setpoint value and provide the control signal 35 to the dosing unit 15. The dosing unit 15 shall dose the disinfectant responsive to the control signal 35. In an aspect, the dosing setpoint value can be determined based on a flow rate of the water. Thus, as illustrated in the example of FIG 1, the system 10 comprises a flow sensor 36 adapted to determine the flow rate of the water and to output a flow signal 37 indicative of the flow rate of the water. The controller 25 is configured to receive the flow signal 37 and determine the dosing setpoint value proportional to the detected flow rate. According to an aspect herein, the controller 25 can be configured to modify the determined dosing setpoint value responsive to the concentration of chlorite detected in the water and the control signal 35 can be generated based on the modified dosing setpoint value. Advantageously, the controller is configured to modify the dosing setpoint value within the predefined range of dosing of the disinfectant into the water. Thus, the modified dosing setpoint value within the predefined range will be the optimized dosing setpoint value. In the present aspect, the controller 25 can be configured to modify the dosing setpoint value based on the comparison of the value of the first signal 30 with the chlorite threshold value. Thus, modifying of the dosing set point value depending on the concentration of chlorite in the water allows maintaining the concentration of chlorite in the water within permissible limits. Additionally, the modification of the dosing setpoint value based on the concentration of chlorite in the water, enables controlling the chlorite concentration in water in the standard process of dosing of disinfectant in the water.

Referring still to FIG 1, in an aspect, an amount of the disinfectant being dosed into the water by the dosing unit 15 can be provided as a feedback to the controller 25 and the controller can be configured to compare the modified dosing setpoint value and the amount of disinfectant being dosed into the water and provide the control signal 35 to the dosing unit 15 based on the difference between the dosing setpoint value and the amount of the disinfectant being dosed into the water. For example, to achieve this, the system 30 can comprise a second sensor 40 adapted to detect a concentration of the disinfectant in the water and output a second signal 45 indicative of the concentration of the disinfectant in the water. The controller 35 can be configured to receive the second signal 45 and can generate the control signal 35 responsive to the difference between the modified dosing setpoint value and the concentration of the disinfectant in the water indicated by the second signal 45. This achieves in correcting the deviations of dosing of the disinfectant into the water with respect to the modified dosing setpoint value more efficiently as the dosing of the disinfectant into the water is controlled using a feedback in a closed loop.

In the present aspect, advantageously, the controller 25 can be configured to modify the dosing setpoint value first signal 30 and the second signal 45. The control signal can be generated by obtaining the values of the first signal 30 and the second signal 45. The value of the second signal 45 provides the concentration of the disinfectant in the water. In an aspect, the controller 25 responsive to the first signal 30 and the second signal 45 can compare the value of the first signal 30 with the chlorite threshold value and the value of the second signal with a predefined range, wherein the predefined range specifies the permissible concentration of disinfectant in the water. The permissible concentration of a disinfectant can be specified by each country for its jurisdiction or can be the one specified by a standard organization. Based on the comparisons, the controller 25 can be configured to generate the control signal 35 such that the concentration of chlorite in the water does not exceed the chlorite threshold value and the concentration of the disinfectant in the water is within the predefined range. For example, if the concentration of chlorite is relatively very less than the chlorite threshold value and the concentration of disinfectant in the water is on the lower side of the predefined range, the control signal 35 can be generated such that the dosing of the disinfectant in the water can be increased to achieve adequate concentration of the disinfectant in the water. Adequate concentration of the disinfectant in the water enables disinfecting the water adequately. The adequate concentration of the disinfectant is achieved by maintaining the concentration of chlorite in the water within the permissible limits. This enables in preventing the users of the water to exposure of concentrations of chlorite above the permissible limits.

A "controller" as used herein is a device for executing machine-readable instructions stored on a computer readable medium, for performing tasks and may comprise any one or combination of, hardware and firmware. For example, the controller may be implemented using a processor, microcontroller, microprocessor, electronic devices, or other electronic units to perform the functions described herein or a combination thereof. The machine-readable instructions may be stored within the controller or external to the controller.

Still referring to FIG 1, in another aspect, the system 10 can further comprise one or more additional sensors for detecting one or more additional parameters influencing the formation of chlorite in the water. One of the parameter influencing the formation of chlorite in the water is the concentration of the disinfectant in the water. The additional parameters can include a temperature of the water, a pH value of the water and duration of the water treatment process. For determining the duration of the treatment process, the controller 25, for example, can be configured to start a timer algorithm from the moment the dosing unit 15 starts dosing the disinfectant. The moment at which the disinfectant is added into the water can be provided as an input from a user. Alternatively, as the controller 25 is coupled to the dosing unit 15, the indication of the moment of dosing the disinfectant into the water can be provided by the dosing unit 15 to the controller 25.

Referring still to FIG 1, according to an embodiment, the system 10 can further comprise a third sensor 50 adapted to detect a temperature of the water and output a third signal 55 indicative of the temperature. The controller 25 can be configured to receive the third signal 50 and generate the control signal 35 responsive to the values of the first signal 30 and the third signal 55. As the temperature of the water is a parameter influencing the formation of chlorite in the water, controlling the dosing of the disinfectant into the water responsive to the concentration of chlorite and the temperature of the water enables in maintaining the concentration of chlorite within the permissible limit more efficiently.

Still referring to FIG 1, in yet another aspect, the system 10 can further comprise a fourth sensor 60 adapted to detect a pH value of the water and output a fourth signal 65 indicative of the pH value of the water. The controller 25 can be configured to receive the fourth signal 65 and generate the control signal 35 responsive to the values of the first signal 30, the third signal 55 and the fourth signal 65. As the pH value of the water is a parameter influencing the formation of chlorite in the water, controlling the dosing of the disinfectant into the water responsive to the concentration of chlorite, temperature of the water and the pH value of the water enables in maintaining the concentration of chlorite within the permissible limit more efficiently. In the shown example of FIG 1, it is illustrated that additional parameters, such as duration of water treatment process, temperature of the water and pH value of the water are determined. However, it should be apparent that one or more of the additional parameters can be detected for controlling the dosing of the disinfectant such that the concentration of chlorite in the water is maintained with the permissible limit more efficiently. By taking into consideration more number of additional parameters, more efficient control of concentration of chlorite within the permissible limit can be obtained.

In embodiments where the dosing setpoint value is determined based on the flow rate of the water, the dosing set point value can be modified based on the concentration of chlorite in the water and one or more of the additional parameters detected and the control signal 35 can be generated based on the modified dosing set point value. Moreover in embodiments, where the system 10 operates in a closed feedback loop for correcting deviations in dosing of the disinfectant in the water, the control signal 35 can be generated based on the difference between the modified dosing setpoint value and the measured concentration of disinfectant in the water.

Referring still to FIG 1, in an aspect, the controller 25 can be configured to generate a look-up table comprising the concentration of chlorite in the water, the concentration of the disinfectant in the water and values of one or more of the additional parameters influencing the formation of chlorite detected at a plurality of measurement cycles. The generated look-up table can be stored at a memory by the controller 25. The memory can be internal memory of the controller 25 or can be an external memory operably coupled to the controller 25. For example, the look-up table generated can be a multidimensional table. This enables in storing the concentration of chlorite in the water based on historical information of the parameters influencing the formation of chlorite. Subsequently, the dosing of the disinfectant in the water can be controlled with reference to the look-up table. As the look-up table is generated using the concentrations of chlorite based on previously detected parameters of the water, the dosing of the disinfectant into the water can be controlled more effectively such that the concentration of the disinfectant in the water is within the predefined range and the concentration of chlorite in the water does not exceed the chlorite threshold value. Additionally, as the previously detected values of the parameters and the concentration of chlorite reflect the control operation performed by the controller 25 with respect to the water, generating the control signal 35 based on previously detected values enables in more efficient control in the dosing of the disinfectant such that the concentration on the disinfectant is maintained within the predefined range more accurately as the influence of the parameters on the formation of chlorite is taken into account more adequately.

FIG 2 illustrates an example of a multidimensional table according to an embodiment herein. The multidimensional table of FIG 2 is generated for the concentration of chlorite in the water, the concentration of the disinfectant in the water and the pH value of the water. In the example of FIG 2, the multidimensional table is generated by storing the pH value of the water for each unit concentration of chlorite in the water and concentration of the disinfectant in the water. For example, the table is created such that the left most column depicts the concentration of chlorite and the rest of the columns depict the ph value of the water for each unit concentration of the disinfectant in the water. The left most column can comprise multiple rows depicting each unit concentration of chlorite and the table can be created by storing the detected pH value of the water in the respective row and column corresponding to the concentration of chlorite detected and the concentration of the disinfectant. In an aspect, the table can be generated for each unit concentration of chlorite and concentration of disinfectant in the water and the detected pH value of the water for the respective concentration of chlorite and concentration of disinfectant can be filled in the respective cell of the column. For example, if the detected concentration of chlorite is 0.02 mg/l and the concentration of the disinfectant is 0.04 and the pH value detected is 6.2, the cell corresponding to the row having the concentration of chlorite depicting 0.02 mg/l and the column depicting the concentration of the disinfectant as 0.04mg/l can be filled by storing the pH value of 6.2.

Referring still to FIG 2, the multidimensional table is illustrated for one additional parameter, i.e., the pH value of water for example purposes only. Multidimensional tables comprising more than one additional parameter can be generated in a similar manner. According to another embodiment, different multidimensional tables can be generated for each unit concentration of the disinfectant in the water. For example, the multidimensional tables can be generated for each unit concentration of the disinfectant detected in the water and then filling out the columns of the table with the respective detected concentration of chlorite and the pH value for that particular concentration of the disinfectant.

Referring now to FIG 1 and FIG 2, the controller 25 can be configured to modify the dosing setpoint value based on the concentration of the disinfectant from the look-up table for the corresponding concentration of chlorite and the pH value of the water. The control signal 35 can be generated responsive to the modified dosing setpoint value. In the shown example of FIG 2, if the detected concentration of chlorite in the water is 0.02 mg/l and the detected pH value is 5.8, the dosing setpoint value can be modified as the concentration of the disinfectant depicted by the respective column, i.e., 0.3 mg/l. Accordingly, the control signal 35 can be generated by the controller 35 in response to the dosing setpoint value modified based on the look-up table.

Still referring to FIG 1, the sensors 20, 40, 50, 60 output the respective signals 30, 45, 55, 65 at respective measurement cycles. Thus, the controller 25 is updated with the detected values at each measurement cycle. Thus, the dosing of the disinfectant into the water can be controlled responsive to the detected values at each measurement cycle in a continuous manner. This enables in controlling the dosing of disinfectant into the water continuously.

Referring still to FIG 1, in an aspect, the controller 25 can be configured to compute a value of quality of the water as a ratio between the detected concentration of the disinfectant and the concentration of chlorite in the water. Additionally, the ratio can be computed by taking into account the additional parameters, such as, temperature of the water and the pH value of the water. The controller 25 can be configured to provide an output signal 70 indicative of the value of quality of the water. A display 75 operably coupled to the controller 25 can be configured to receive the output signal 70 and provide a visual indication of the value of quality of the water. For example, the value of quality of the water can be computed for each of the measurement cycles of the sensors 20, 40 and the display can be refreshed to provide the visual indication of the value of quality of the water at for each of the measurement cycle.

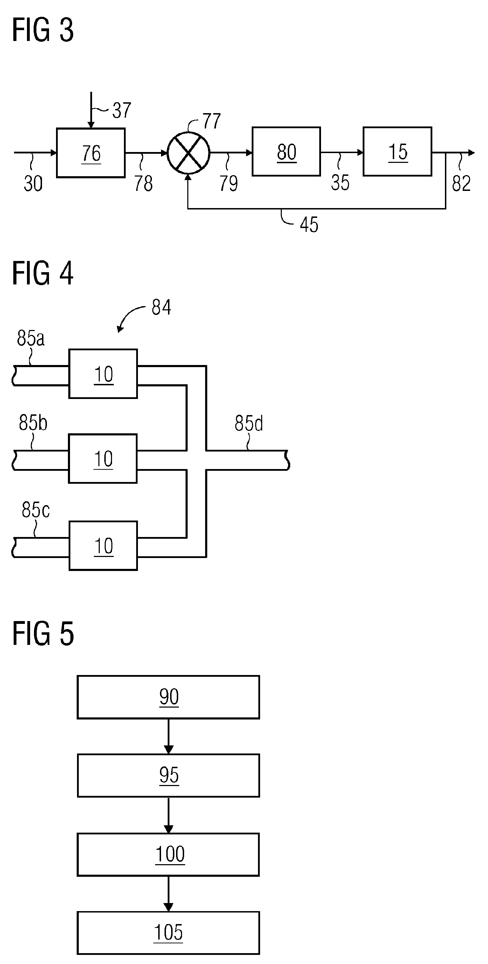

FIG 3 illustrates an exemplary block diagram of a simplified single feedback closed loop control for controlling the dosing of the disinfectant according to an embodiment herein. The setpoint controller 76 is configured to receive the first signal 30, the flow signal 37. The setpoint controller 76 is configured to determine the dosing setpoint value based on the flow rate of the water and modify the determined dosing setpoint value responsive to the first signal 30 such that the concentration of chlorite does not exceed the chlorite threshold value. The comparator 77 is configured to receive the modified dosing setpoint value 78 and the second signal 45 and output an error signal 79 based on the difference between the modified dosing setpoint value and the concentration of the disinfectant in the water. The disinfectant controller 80 is configured to receive the error signal 79 and output the control signal 35 to be provided to the dosing unit 15 for dosing of the disinfectant into the water. The dosing unit 15 doses the disinfectant into water, designated as 82. The disinfectant dosed into the water is provided as a feedback to the comparator 78 via the second signal 45 which indicates the concentration of disinfectant in the water. According to the aspects herein, the controller 25 of FIG 1 is configured to perform the functions of the setpoint controller 76, comparator 78, and the disinfectant controller 80.

FIG 4 illustrates a schematic diagram of a water distribution system 84 comprising a plurality of systems 10 of FIG 1 for controlling dosing of a chlorine dioxide based disinfectant in water according to an embodiment herein. The water distribution system 84 is illustrated as comprising a plurality of pipes 85a, 85b, 85c fluidly connected to a main pipe 85d for distribution. Each of the pipes 85a, 85b, 85c are associated with a respective system 10. The arrangement of pipes 85 and the respective system 10 in FIG 4 is illustrated by way of example only and the pipes 85 and the systems 10 may be arranged in different manner. The water can be distributed for domestic usage, industrial usage and the like. Thus, the water is transported to the end user via the pipes 85. The pipes 85a, 85b, 85c can be used for bringing water from different locations or sources and thus the water quality in the pipes 85a, 85b, 85c can differ. In such distributions systems 84, disinfectant is dosed into the water in the pipes 85a, 85b, 85c based on the flow rate of the water in the respective pipes 85a, 85b, 85c. Dosing of the disinfectant into the pipes allows for correction of dosing of the disinfectant into the water. Accordingly, the system 10 comprising the sensors 20, 36, 40, 50, 60, the controller 25 and the dosing unit 15 can be arranged along each of the pipes 85a, 85b, 85c such that the sensors 20, 36, 40, 50, 60 are in fluid communication with the water to detect the respective parameters.

Referring still to FIG 4, the controller 25 is configured to determine the dosing setpoint value based on the flow rate of the water in the respective pipes 85a, 85b, 85c and modify the determined dosing setpoint value based on the concentration of chlorite in the water in the respective pipes 85a, 85b, 85c. The dosing setpoint value can also be modified based on one or more of the additional parameters. The control signal 35 to be provided to the dosing unit 15 for dosing of the disinfectant is generated based on the modified dosing setpoint value. In an aspect, each system 10 can comprise a plurality of sensors 20, 37, 40, 50, 60 and the same can be positioned at multiple locations along the respective pipes 85a, 85b, 85c and the controller 25 can be configured to modify the dosing setpoint value on the basis of either the highest or the lowest values of the respective signals as per the dependency of formation of chlorite on the respective parameters. For example, the dosing setpoint value can be modified responsive to the one of the first signals 30 indicating the highest concentration of chlorite in the water in the respective pipes 85a, 85b, 85c. As the first sensors 20 are arranged at multiple locations along the pipes 85a, 85b, 85c, the concentration of chlorite detected by the first sensors 20 may differ at different locations with the respective pipes 85a, 85b, 85c as there is further reaction happening in the pipes 85a, 85,b, 85c due to more duration of reaction of the disinfectant with the water.. Thus, controlling the dosing of the disinfectant into the water responsive to the highest concentration of chlorite detected enables in controlling the concentration of chlorite in the watermore effectively.

Referring still to FIG 4, according to an embodiment, the control signal 35 can be generated by computing a respective average amount of the values indicated by the first signals 30, the second signals 45, the third signals 55 and the fourth signals 65 provided by two or more of the first sensors 20, the second sensors 40, the third sensors 50 and the fourth sensors 60 along the respective pipes 85a, 85b, 85c. This enables in controlling the dosing of the disinfectant into the water in the respective pipes 85a, 85b, 85c more effectively as the parameters influencing the formation of chlorite are detected at multiple locations, and the value of the parameter influencing the formation of chlorite can be considered more adequately. According to an embodiment, the respective controller 25 and the respective systems 10 can be configured to compute the water quality in the respective pipes 85a, 85b, 85c. The value of water quality computed can be compared with a threshold value and based on the comparison the controller can be configured to stop the influent of water of bad quality into the main pipe 85d. Alternatively, the water quality computed can be provided to a central controller and the central controller can be configured to control the influent of water of bad quality into the main pipe 85d. The controllers 25 of the systems 10 and the central controller can be connected wireless or via wire as desired.

FIG 5 with reference to FIG 1 through FIG 4, is a flow diagram illustrating a method of controlling dosing of a chlorine dioxide based disinfectant into water according to an embodiment herein. At block 90, at least one dosing unit 15 is provided for dosing the disinfectant into the water. Next, at block 95, a concentration of chlorite in the water is detected. Moving next to block 100, a control signal (35) is generated responsive to the concentration of chlorite detected. Next, at block 105, the at least one dosing unit 15 is controlled for dosing the disinfectant into the water responsive to the control signal (35) such that the concentration of chlorite does not exceed a chlorite threshold value.

The embodiments described herein can be used for maintaining the concentration of chlorite in water within the permissible limits generated due to addition of chlorine dioxide based disinfectants to water. Water can be disinfected for human consumption, such as, swimming, bathing, drinking, and the like and also for industrial use, e.g. in water coolant circuits. Chlorite is one of the by-products generated when water is disinfected with specifies of chlorine. Chlorite has negative effect on the health of a user when the user is exposed to concentrations of chlorite above the permissible limits. Thus, controlling the dosing of the disinfectant into the water responsive to the concentration of chlorite in the water enables in maintaining the concentration of chlorite in the water within the permissible limits more effectively. The embodiments described can be implemented with a standard process of dosing of disinfectant into water which are well established and recognized. Additionally, the dosing of the disinfectant into the water can be controlled in a continuous manner without requiring stopping of the dosing of the disinfectant into the water. Moreover, drinking water is supplied in pipes and the embodiments described herein enables in efficient control and correction of dosing of disinfectant into the water such that concentration of chlorite is maintained within the permissible limits.

While this invention has been described in detail with reference to certain preferred embodiments, it should be appreciated that the present invention is not limited to those precise embodiments. Rather, in view of the present disclosure which describes the current best mode for practicing the invention, many modifications and variations would present themselves, to those of skill in the art without departing from the scope and spirit of this invention. The scope of the invention is, therefore, indicated by the following claims rather than by the foregoing description. All changes, modifications, and variations coming within the meaning and range of equivalency of the claims are to be considered within their scope.

## Claims

1. A system (10) for controlling dosing of a chlorine dioxide based disinfectant into water, comprising:
- at least one dosing unit (15) for dosing the disinfectant into the water,
- at least one first sensor (20) adapted to detect a concentration of chlorite in the water and configured to output respective first signals (30) indicative of the concentration of the chlorite, and
- a controller (25) operably coupled to the at least one first sensor (20) to receive the first signals (30) and operably coupled to the at least one dosing unit (15) to send a control signal (35) to the at least one dosing unit (15) in response to the at least one of the first signals (20) such that the concentration of chlorite does not exceed a chlorite threshold value.

2. The system (10) according to claim 1, further comprising at least one second sensor (40) adapted to detect a concentration of the disinfectant in the water and configured to output respective second signals (45) indicative of the concentration of the disinfectant, wherein the controller (25) is configured to receive the respective second signals (45) and configured to generate the control signal (35) further responsive to the at least one of the second signals (45) such that the concentration of disinfectant in the water is equal to a modified dosing setpoint value (78), wherein the modified dosing setpoint value (78) is obtained by modifying a dosing setpoint value responsive to the at least one of the first signals (30).

3. The system (10) according to claim 1 or claim 2, further comprising one or more additional sensors (50, 60) adapted to detect one or more additional parameters influencing the formation of chlorite in the water.

4. The system (10) according to claim 3, wherein the additional parameter(s) include(s) a temperature and/or a pH value of the water.

5. The system (10) according to claim 3 or claim 4, wherein the controller (25) is further configured to generate a look-up table comprising the detected concentration of chlorite, the concentration of disinfectant in the water, and values of at least one of the additional parameter of a plurality of respective measurement cycles and configured to store the generated look-up table in a memory.

6. The system (10) according to claim 5, wherein the controller (25) is further configured to generate the control signal (35) in response to the concentration of disinfectant from the look-up table for the detected concentration of chlorite in the water and value of at least one of the additional parameter such that the concentration of chlorite does not exceed the chlorite threshold value.

7. The system (10) according to anyone of claims 1 to 6, wherein the controller (25) is configured to generate the control signal (35) in response to a highest concentration of chlorite in the water or an average amount of the concentration of chlorite in the water indicated by the respective first signals (30) provided by two or more of the first sensors (20) at the same time.

8. The system (10) according to anyone of claims 1 to 7, wherein the controller (25) is further configured to compute a value of quality of the water as a ratio of the detected concentration of the disinfectant in the water to the concentration of chlorite in the water and provide an output signal (70) indicative of the value of quality of the water.

9. The system (10) according to claim 8, further comprising a display (75) operably coupled to the controller (25) and configured to receive the output signal (70) and provide a visual indication of the value of quality of the water responsive to the output signal (70).

10. A method of controlling dosing of a chlorine dioxide based disinfectant into water, the method comprising:
- providing at least one dosing unit (15) for dosing the disinfectant into the water,
- detecting a concentration of chlorite in the water,
- generating a control signal (35) responsive to the concentration of chlorite detected, and
- controlling the at least one dosing unit (15) for dosing the disinfectant into the water responsive to the control signal (35) such that the concentration of chlorite does not exceed a chlorite threshold value.

11. The method according to claim 10, wherein the generating of the control signal (35) includes:
- detecting a concentration of the disinfectant in the water, and
- generating the control signal (35) responsive to the concentration of disinfectant in the water such that the concentration of disinfectant in the water is equal to a modified dosing setpoint value, wherein the modified dosing setpoint value is obtained by modifying a dosing setpoint value responsive to the concentration of chlorite in the water.

12. The method according to claim 9 or claim 10, wherein the generating of the control signal (35) includes:
- detecting at least one additional parameter influencing the formation of chlorite in the water,
- generating a look-up table comprising the detected concentration of chlorite, the concentration of disinfectant in the water, and values of at least one of the additional parameters of a plurality of respective measurement cycles, and
- storing the generated look-up table in a memory.

13. The method according to claim 12, wherein the control signal (35) is generated in response to the concentration of the disinfectant from the look-up table for the detected concentration of chlorite and the value of at least one of the additional parameters.

14. The method according to anyone of claims 10 to 13, wherein the control signal (35) is generated in response to a highest concentration of chlorite in the water or an average amount of the concentration of chlorite in the water indicated by the respective first signals (30) provided by two or more of the first sensors (20) at the same time.

15. The method according to anyone of claims 11 to 14, further comprising:
- computing a value of quality of the water as a ratio of the detected concentration of the disinfectant in the water to the concentration of chlorite in the water, and
- providing a visual indication of the value of quality of the water.
